# EUROPÄISCHE PATENTSCHRIFT

(11) **EP 1 356 146 B1**
(45) Veröffentlichungstag und Bekanntmachung des Hinweises auf die Patenterteilung: **21.02.2007**
(21) Anmeldenummer: 01985900.8
(22) Anmeldetag: 18.12.2001
(51) Int. Cl.: D04H 1/46, D04H 13/00, D06C 23/00

(54) **VERFAHREN ZUR HYDRODYNAMISCHEN BEAUFSCHLAGUNG EINER GGF. AUCH MIT ENDLICHEN VORPRODUKTEN VERSEHENEN WARENBAHN MIT WASSERSTRAHLEN UND DÜSENEINRICHTUNG ZUR ERZEUGUNG VON FLÜSSIGKEITSSTRAHLEN**
METHOD FOR HYDRODYNAMICALLY SUBJECTING A GOODS LINE, OPTIONALLY WITH FINITE PRE-PRODUCTS, TO WATER JETS AND NOZZLE DEVICE FOR PRODUCING LIQUID JETS
PROCEDE HYDRODYNAMIQUE PERMETTANT D'EXPOSER UNE BANDE EVENTUELLEMENT DOTEE DE DEMI-PRODUITS FINIS, A DES JETS D'EAU ET DISPOSITIF A BUSES DESTINE A LA PRODUCTION DE JETS DE LIQUIDE

(30) Priorität: 22.12.2000 DE 10064687
(43) Veröffentlichungstag der Anmeldung: 29.10.2003
(73) Patentinhaber: Fleissner GmbH, 63329 Egelsbach (DE); Vliesstoff-Technologie in 3. Dimension KG, 56566 Neuwied (DE)
(72) Erfinder: BARTH, Martin, 56579 Rengsdorf (DE); WATZL, Alfred, 63322 Rödermark (DE); MÜNSTERMANN, Ullrich, 63329 Egelsbach (DE); FECHTER, Thomas, 63456 Hanau (DE)
(74) Vertreter: Meyer-Dulheuer, Karl-Hermann
(86) Internationale Anmeldenummer: PCT/EP2001/014973
(87) Internationale Veröffentlichungsnummer: WO 2002/052083

(56) Entgegenhaltungen:
- EP-A- 0 132 028
- EP-A- 0 177 277
- EP-A- 0 333 210
- EP-A- 0 445 908
- EP-A- 0 598 559
- EP-A- 0 703 308
- EP-A- 0 725 175
- EP-A- 0 795 916
- EP-A- 1 005 845
- WO-A-02/48441
- US-A- 3 508 308
- US-A- 4 783 977
- US-A- 4 984 340
- US-A- 5 235 733
- PATENT ABSTRACTS OF JAPAN vol. 2000, no. 01, 31. Januar 2000 (2000-01-31) & JP 11 280655 A (MARUYAMA MFG CO LTD), 15. Oktober 1999 (1999-10-15)
- PATENT ABSTRACTS OF JAPAN vol. 017, no. 655 (C-1136), 6. Dezember 1993 (1993-12-06) & JP 05 209360 A (DAIWABO CREATE KK), 20. August 1993 (1993-08-20)
- PATENT ABSTRACTS OF JAPAN vol. 1998, no. 01, 30. Januar 1998 (1998-01-30) & JP 09 241958 A (KASEN NOZURU SEISAKUSHO:KK), 16. September 1997 (1997-09-16) & JP 09 241958 A (KASEN NOZURU SEISAKUSHO KK.) 16. September 1999 (1999-09-16)
- PATENT ABSTRACTS OF JAPAN vol. 014, no. 578 (C-0791), 21. Dezember 1990 (1990-12-21) & JP 02 251695 A (ORIBESUTO KK), 9. Oktober 1990 (1990-10-09)

## Beschreibung

Durch die WO 00/63479 ist es bekannt, auch dreidimensionale, endliche Güter wie auch Vor oder Zwischenprodukte zwischen zwei Warenbahnen wie Vliese zu lagern, durch hydrodynamische Vernadelung die Vliese durch Verfilzen derer Fasern zu verbinden, zu verfestigen und dadurch die Güter auch rundum zu verschließen.

Es ist ferner eine Vorrichtung bekannt (WO 02/48441) bekannt, wobei diese Vorrichtung mit mehreren Düsenbalken oder Düsen im Gehäuse an einer Vorrichtung zur Erzeugung von Flüssigkeitsstrahlen zur Strahlbeaufschlagung der Fasern von zumindest zwei entlang des Balkens mittels einer Trommel oder eines Endlosbandes geführten Bahnen, wie Faserbahn, Tissue, Gewebe oder Gewirke, der oder die aus einem sich über einen Teil der Arbeitsbreite der Bahn erstreckenden oberen Teil und einem unteren Teil besteht, wobei in dem oberen Teil eine Druckkammer angeordnet ist, der die unter Druck stehende Flüssigkeit zugeführt ist, und an dem Unterteil ein Düsenblech mit den Öffnungen für die Düsen flüssigkeitsdicht gelagert ist.

Solange der Verfestigungsvorgang fortlaufend über die Länge der vorlaufenden Sandwichbahn erfolgen soll, - gleichgütig ob nur die dreidimensionalen Güter parallel zur Transportrichtung der Warenbahn verlaufen oder senkrecht dazu - besteht kein Unterschied zum vorbekannten Verfestigungsverfahren mittels der Vernadelung nach z. B. der US-A-3 508 308. Sind jedoch die Güter endlich und sollen sie nicht oder nur teilweise im Bereich der Güter von den Wasserstrahlen getroffen werden, so können die Güter mit den vorbekannten Verfahren mit der Wasservernadelung nicht teilweise über die Fläche verfestigt oder rundum eingepackt werden.

Der Erfindung liegt die Aufgabe zugrunde, ein Verfahren und dazu eine Vorrichtung zu finden, mit dem auch solche endliche Güter wie bereits vorgefertigte Polster- und/oder Saugeinlagen für Windeln, Wundauflagen, Kompressen, evtl. auch Pflaster od. dgl. Fertigprodukte kontinuierlich, aber auch ggf. teilweise über ihre gesamte Oberfläche hinweg verfestigt, teilweise rundum unter Aussparung der Fläche der Güter in die ggf. beiden oben und unten vorlaufenden Vliese oder dgl. gebunden werden können, wodurch die Güter rundum eingeschlossen oder eingesiegelt werden. Die Endprodukte für Hygiene, Medizin oder andere technische Anwendungen sollen kontinuierlich, aber gezielt auf das jeweilige auch dreidimensionale Produkt unterschiedlich über die Fläche behandelt werden können.

Es sind aber auch Fälle denkbar, in denen ein flächiges Vlies oder Vliesprodukt nur teilweise über die Fläche zu verdichten und demgemäß auch zu verfestigen ist, oder um spezielle Effekte über die Fläche zu erzielen. Insofern sollte das zu findende Verfahren auch nutzbar sein, um diese nur teilweise Verfestigung des Vlieses oder des Verbundmaterials zu gestalten.

Ausgehend von einem Verfahren zum hydrodynamischen Verdichten und/oder Verfestigen und/oder Verbinden im Falle von zumindest zwei aufeinander liegenden Vliesen, Tissue, Geweben oder Gewirken mit gleichmäßig über die Arbeitsbreite wirksamen Flüssigkeitsstrahlen, indem mit hohem Druck von bis zu 1000 bar eine Flüssigkeit aus feinen, in Reihe dicht beieinander angeordneten Düsenöffnungen aus einem über die Arbeitsbreite sich erstreckenden Düsenstreifen eines Düsenbalkens gegen die gegenüber dem Düsenbalken vorlaufende Warenbahn gespritzt wird, sieht die Erfindung zur Lösung der gestellten Aufgabe vor, dass bei unverändert aus dem Wasserbalken fortlaufend austretenden Flüssigkeitsstrahlen diese kurzzeitig gehindert werden, die vorlaufende Warenbahn zu treffen, um Teile, Linien, Flächen der Warenbahn von den Wasserstrahlen unbeaufschlagt zu lassen. Die Wasserstrahlen können oder der Wasserdruck kann auch unverändert in dem Wasserbalken erzeugt und dann die Wasserstrahlen zumindest teilweise gehindert werden, die Warenbahn zu erreichen. Dies ist beispielsweise möglich, indem die Wasserstrahlen aus ihrer Erzeugungsrichtung kurzzeitig abgelenkt werden, indem in die Strahlrichtung der Wasserstrahlen ein Gegenstand zeitlich definiert bewegt wird, der dann folgerichtig die Bewegungsbahn der Wasserstrahlen ggf. für einen kurzen Augenblick verändert. Auf diese Weise können Produkte auch in Bereichen unbehandelt belassen bleiben, wo sie voluminös verbleiben sollen, um z. B. das spezifische Saugverhalten der vorhandenen Produkte zu erhalten.

Ein ähnlicher Effekt lässt sich auch dadurch erzielen, dass einzelne Düsen, Gruppen von Düsen oder einzelne Segmente eines breiteren Düsenbalkens intermittierend mit Flüssigkeit versorgt werden bzw. solche abgeben, was z. B. durch elektromechanisch, elektromagnetisch oder piezoelektrisch angesteuerte Ventile oder Ventilgruppen (parallel oder ggf. sequentiell) erreicht werden kann. Es ist dadurch möglich, pulsierende Flüssigkeitsstrahlen in situ, d. h. gezielt auf einzelne, genau definierte Bereiche des hydrodynamisch zu beaufschlagenden Materials zu richten und dadurch eine Befestigung oder Verfestigung nach einem vorgegebenen Muster zu erreichen.

Dies ist gleichartig möglich, wenn der in die Bewegungsbahn der Wasserstrahlen hinein sich bewegende Gegenstand Teil eines mit der Warenbahn sich bewegenden Bandes oder der Umfangsfläche einer Trommel ist. Hier ist nur zu beachten, dass die in dem Band oder an der Trommel notwendige Fläche irgendwie seitlich geführt oder gehalten und damit vorwärtsbewegt werden kann. Folgerichtig muss dieser flüssigkeitsdurchlässige Bereich, wie Sieb oder gelochtes Blech, jedenfalls teilweise entlang der Strahlen der Flüssigkeit einen Widerstand entgegenstellen. Dies führt zu einem beliebigen Muster wie auch Gewebe- oder Waffelmuster in der Verfestigungszone. Anwendungsbeispiele sind vielfältig. Es kann Papier mit einer Art Wasserzeichen oder es kann ein Tissue mit einer für die Herstellerfirma oder deren Kunden spezifischen Oberflächenstruktur versehen werden. Typisch sind hier auch Watteprodukte, die ein- oder beidseitig mit einer leichten Oberflächenstrukturierung versehen werden. die eine Linienvertiefung hat, also ein Netz, parallele Linien gerade oder hin- und hergehender Art.

Eine andere Möglichkeit die gestellte Aufgabe zu erfüllen besteht darin, dass der Düsenbalken oder die Düsen in Bewegungen relativ zur Warenbahn oder umgekehrt gesteuert wird oder werden. Dabei kann auch gleichzeitig der Austritt des Wassers aus der Düse oder dem Düsenbalken in Druck und/oder Menge in kurzen Abständen gesteuert werden. Für die Verwirklichung dieser Idee sind unterschiedliche Methoden denkbar. Es können einzelne Düsen verwendet werden, die mit der Druckpumpe zur Herstellung des Wasserdruckes in Verbindung stehen. Es kann aber auch ein üblicher Düsenbalken verwendet werden, an den Einzeldüsen angeschlossen sind, die wiederum mit Ventilen versehen sind, die über z. B. Computer ein besonderes Muster mit Wasserstrahlen bearbeiten oder eben nicht bearbeiten. Auf diesem Wege kann auch gezielt die Beaufschlagung des zu verfestigenden Materials mit Flüssigkeitsstrahlen gemindert oder auf einzelne Teilflächen, Punkte oder Linien konzentriert werden. Das setzt ein Intermittieren oder Pulsieren der Strahlen voraus.

Mit diesen punktförmigen oder kurzen strichförmigen Vernadelungen kann eine Art hydrodynamisches Nähen, Heften oder Tacking erzielt werden, und zwar ohne thermoplastisches Verfestigen des Vliesproduktes, was die angestrebte Saugwirkung des Produktes schmälern und dessen Weichheit und Anschmiegsamkeit beeinträchtigen würde. Die Flexibilität auf diesem Gebiet ist enorm. In jegliche Formate oder Muster kann verfestigt werden. Das System kann insbesondere für die Hygieneindustrie verwendet werden.

Auf diese Weise können bisher einzeln gefertigte Vorprodukte jetzt kontinuierlich fertig behandelt, verfestigt, in Hüllen eingeschlossen werden. Die Produktionsbahn kann wie üblich mit diesen diskontinuierlichen Produkten nach Trocknung und/oder weiterer Veredelung zu großen Rollen aufgewickelt und versandt werden und vor Ort dann nach Quer- und Längsteilung zur einzelnen Verwendung wie auch Verpackung in Kartons usw. gelangen.

In der Zeichnung sind mehrere Beispiele von Vorrichtungen zur Durchführung der Verfahren nach der Erfindung schematisch dargestellt. Es zeigen:
- Fig. 1: eine Draufsicht auf eine Warenbahn mit plastisch erhöhten, in Abstand nebeneinander angeordneten Gütern,
- Fig. 2: eine Seitenansicht von Wasserbalken, Einschubblech und Warenbahn,
- Fig. 3: eine Seitenansicht wie Fig. 2 mit der Möglichkeit einer punktförmigen Verfestigung,
- Fig. 4: steuerbare Einzeldüsen mit Details a) bis d)
- Fig. 5: im Querschnitt ein Düsenbalken innerhalb eines mit der Warenbahn vorlaufenden Endlosbandes, dessen Netzstruktur ggf. auch durch aus
- Fig. 6: ersichtliche undurchlässige Bereiche verschlossen ist.

Ein Düsenbalken besteht grundsätzlich aus den Teilen wie sie in der EP-A-0 725 175 beschrieben sind. Insofern wird auf die Offenbarung dieser Offenlegungsschrift Bezug genommen.

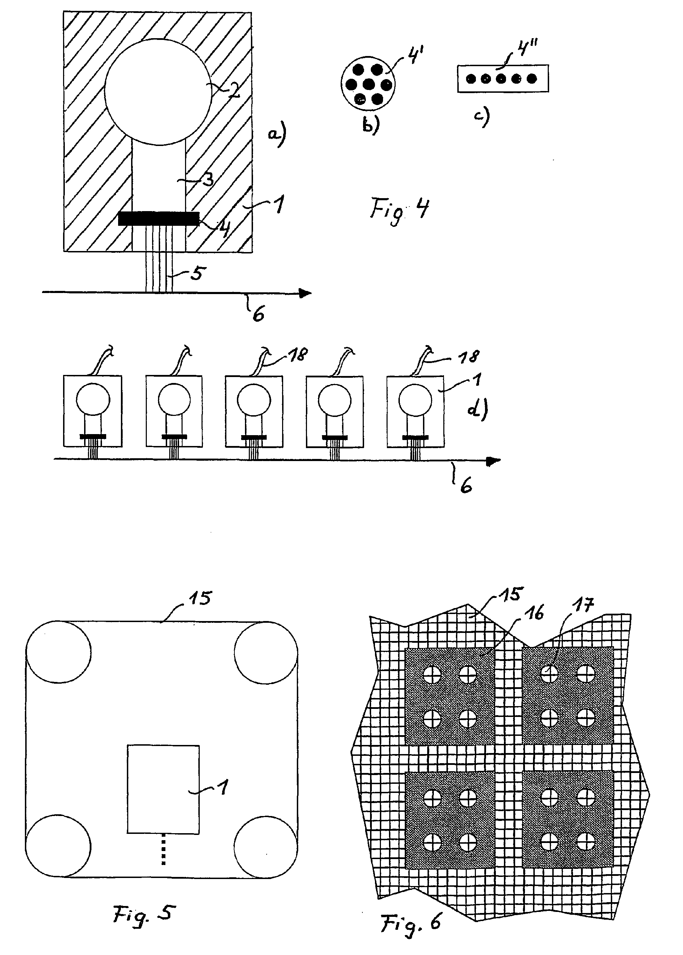

Ein Düsenbalken 1 wie er vereinfacht in Fig. 4a) dargestellt ist, besteht aus einem Gehäuse mit jedenfalls einer Längsbohrung 2, der stirnseitig die unter Druck stehende Flüssigkeit zugeführt ist. Über Durchflussbohrungen 3 gelangt das Wasser zum Düsenstreifen 4, in dem meist in zwei Reihen nebeneinander und auf Lücke Düsenlöcher eingebracht sind. In ihnen bilden sich die Wasserstrahlen 5, die gegen die unten vorbeilaufende Warenbahn 6 aus zumindest einem z. B. Vlies zur Faserverschlingung prallen.

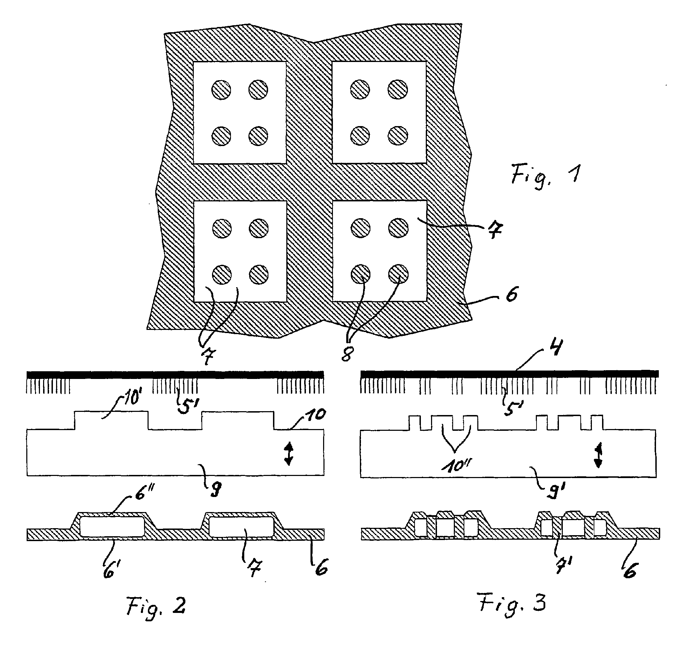

Die Warenbahn 6 besteht gemäß Fig. 1 aus einem unteren Trägervlies 6', auf dem flächenförmige Vorprodukte 7 wie Polster und Saugeinlagen für Windeln, Pflaster, Pads od. dgl. aufgelegt sind. Die dargestellten Gebrauchsgüter 7 sollen hier auch punktförmig verfestigt oder angeheftet werden, weswegen die Punktverfestigung 8 jeweils an vier Stellen eingezeichnet ist. Der übrige Bereich sollte unverändert voluminös und damit hoch saugfähig verbleiben. Auf dieses Gebrauchsgut 7 ist wiederum ein flächenförmiges Deckvlies 6" aufgelegt. Alles miteinander soll durch eine spezielle Wasservernadelung wie durch die Schraffierung angedeutet verbunden werden.

Dazu sind in Fig. 2 und 3 in Ansicht oder Querschnitt jeweils die Wasserstrahlen 5 aus einem hier nicht dargestellten Düsenbalken, einem ggf. hin und her verlagerbaren Gegenstand 9, der in diesem Beispiel an der Vorderkante 10 profiliert eingeschnitten ist, und die Warenbahn 6 im Schnitt dort dargestellt, wo die spezielle Nicht-Verfestigung oder Punktverfestigung erfolgt ist. Durch die partiell wirksam werdenden Wasserstrahlen 5 werden die Gebrauchsgüter 7 oder die Vliese 6' und 6" nur an den gewünschten Stellen von den Wasserstrahlen 5 getroffen. Dazu dient in Fig. 2, 3 der Gegenstand 9, der hier mit Blech bezeichnet werden soll. Das Blech 9 hat eine Vorderkante 10, die gegen die gleichmäßig über die Arbeitsbreite aus dem Düsenbalken austretenden Flüssigkeitsstrahlen 5 hin und her bewegbar ist. An den vorstehenden Profilen 10' gelangt das Blech 9 unter die Wasserstrahlen 5, so dass diese hier auf das Blech 9 auftreffenden Wasserstrahlen nicht auf die darunter her geführte Warenbahn 6 treffen und damit das Fertigprodukt voluminös und dort hoch saugfähig belassen. In Fig. 2 sind die profiliert vorstehenden Abdeckbereiche 10' in der Breite der Gebrauchsgüter 7 dargestellt, weil diese hier nicht von den Wasserstrahlen 5' getroffen werden und damit voluminös bleiben sollen, während in Fig. 3 die Abdeckbereiche 10" des Bleches 9' schmaler, nämlich in der Breite der Stege 7' wegen der Punktverfestigung 8 schmal ausgebildet sind, damit nur an den Punkten 8 die Wasserstrahlen 5' auftreffen und nur dort die Gebrauchsgüter 7 verfestigen.

Bei einer in mehreren Stufen notwendigen Verfestigung wie in Fig. 1 dargestellt sind mehrere Bleche 9, 9' hintereinander mit jeweils einem Wasserbalken vorgesehen. Die einzelnen Bleche 9, 9' werden unabhängig voneinander mit verschiedenen Takten hin und her bewegt.

Es können hier auch differenziert hergestellte Düsenstreifen zur Anwendung gelangen, die über ihre Länge mit unterschiedlich gelochten und/oder nicht gelochten Bereichen im Düsenbalken wirksam sind. Haben z. B. die Düsenstreifen 4 nur dort Löcher, wo die Wasserstrahlen 5' in Fig. 2 und 3 austreten, so genügt ein hin und her sich bewegendes Blech 9 mit gerader Vorderkante 10, um abschnittsweise oder auch nur an Punkten eine Verfestigung im Bereich der Produkte 7 zu bewirken.

Die Düsenstreifen mit über ihre Länge unterschiedlich verteilten Lochungen oder nicht gelochten Bereichen können auch alleine zur abschnittsweisen Verfestigung dienlich sein. So können Muster in das Vlies mit Düsenstrahlen gebracht werden, die der Gestaltung der Düsenstreifen entsprechen.

Bei dem Ausführungsbeispiel nach Fig. 4 sind Einzeldüsen oder kleinere Wasserbalken 1 vorgesehen. Die Einzeldüsen haben hier eine kreisförmige Düsenplatte 4' gemäß Fig. 4b oder gemäß Fig. 4c eine streifenförmige Düsenplatte 4" mit jeweils nur wenig Löchern für die Wasserstrahlen 5. Die Einzeldüsen gemäß Fig. 4d können entlang eines von einem Computer gesteuerten Weges laufen und damit jedes beliebige Muster verfestigen und auf die Warenbahn 6 zeichnen. Die Einzeldüsen können in der zugeführten Flüssigkeitsmenge und in dessen Druck besser gesteuert werden. Mit diesen Düsen ist das Vlies 6 ggf. auch linienförmig zu lochen oder zu heften, auch zum leichteren Trennen der Randabschnitte oder der Gebrauchsgüter 7 voneinander. Die Einzeldüsen können auch zur speziellen Verfestigung der Güter, z. B. auch an den Randbereichen in unterschiedlicher Höhe zur Warenbahn angeordnet oder in speziell angepasster Neigung ausgerichtet sein. Die Einzeldüsen 1 nach Fig. 4d müssen mit dem notwendigen Druckwasser versorgt werden. Entweder sind die Einzeldüsen mit der Druckpumpe über bewegliche Schläuche 18 verbunden oder diese Schläuche 18 sind an einen Wasserbalken üblicher Bauart angeschlossen. Es ist dann über die Breite der Warenbahn ein Wasserbalken 1 angeordnet, an dem eine Reihe von Schläuchen 18 druckdicht befestigt sind, die bis hin zu den Einzeldüsen 1 reichen. Die Düsen können mit Ventilen versorgt sein, die den Wasserdruck von dem Wasserbalken beeinflussen, je nachdem, welcher Druck oder welche Flüssigkeitsmenge an der bestimmten Stelle der Warenbahn 6 benötigt wird. Alles kann über Computer gesteuert werden.

Ein Gebrauchsgut 7 kann mit dem gleichen Muster auch mit der Vorrichtung nach der Fig. 5 verfestigt werden. Dort läuft mit der Warenbahn 6 ein Endlosband 15 und deckt die Warenbahn 6 vollflächig mit einem Sieb ab. Statt eines Endlosbandes kann auch eine Trommel mit großer freier Oberfläche vorteilhaft sein. Auf der Innenseite des Endlosbandes 15 ist der Wasserbalken 1 angeordnet, der über seine ganze Länge gleichmäßig Flüssigkeitsstrahlen gegen das Band spritzt. Das Band 15 hat aber Bereiche 16 die flüssigkeitsundurchlässig sind. Diese Abdeckbereiche 16 entsprechen dem zu vernadelnden Muster z. B. gemäß den aus Fig. 1 entnehmbaren Gebrauchsgegenständen 7 mit Öffnungen 17 für die Punktvernadelung. Auf diese Weise können aber auch andere flächige Muster in Abhängigkeit des mustergebenden Endlosbandes 15 in und auf die Warenbahn 6 genadelt, geprägt werden. Diese Art der Verfestigung bedingt aber ein Muster auch auf den verfestigten Vliesbereichen, das der Art, dem Mustergeflecht des Endlosbandes 15 entspricht.

## Patentansprüche

1. Verfahren zum hydrodynamischen Verdichten und/oder Verfestigen und/oder Verbinden im Falle von zumindest zwei aufeinander liegenden Vliesen, Tissue, Geweben oder Gewirken mit gleichmäßig über eine Arbeitsbreite aus einem Düsenbalken austretenden Flüssigkeitsstrahlen, indem mit Druck von bis zu 1000 bar eine Flüssigkeit aus feinen, in Reihe dicht beieinander angeordneten Düsenöffnungen z. B. aus einem über eine Arbeitsbreite sich erstreckenden Düsenstreifen (4) eines Düsenbalkens gegen die gegenüber dem Düsenbalken vorlaufende Warenbahn gespritzt wird, **dadurch gekennzeichnet, dass** zum selektiven Verfestigen und/oder Verbinden, zum Einschließen einer endlichen, dreidimensionalen, abgedeckten oder mittleren Schicht bei unverändert aus den die Wasserstrahlen erzeugenden Elementen austretenden Flüssigkeitsstrahlen diese teilweise kurzzeitig gehindert werden die vorlaufende Warenbahn, und zwar im Bereich der endlichen mittleren Schicht, zu treffen, um Teile, Linien, Flächen der Warenbahn von den Wasserstrahlen unbeaufschlagt zu belassen, die Randbereiche dieser Flächen jedoch zu vernadeln zum Verbinden der dort nur zwei oder mehr aufeinanderliegenden Vliese.

2. Verfahren nach Anspruch 1, **dadurch gekennzeichnet, dass** bei unverändert in dem Wasserbalken fortlaufend gegen den Düsenstreifen wirkendem Flüssigkeitsdruck dieser über die Länge des Düsenstreifens teilweise gehindert wird, Wasserstrahlen zu erzeugen, um die vorlaufende Warenbahn in diesem Bereich mit der endlichen, dreidimensionalen, abgedeckten oder mittleren Schicht nicht zu treffen, um Teile, Linien, Flächen der Warenbahn von den Wasserstrahlen unbeaufschlagt zu belassen, die Randbereiche dieser Flächen jedoch zu vernadeln zum Verbinden der dort nur zwei oder mehr aufeinanderliegenden Vliese.

3. Verfahren nach Anspruch 1 oder 2, **dadurch gekennzeichnet, dass** in die Strahlrichtung der Wasserstrahlen ein Gegenstand bewegt wird, der die Bewegungsrichtung der Wasserstrahlen verändert.

4. Verfahren nach Anspruch 3, **dadurch gekennzeichnet, dass** der Gegenstand in die Bewegungsrichtung der Wasserstrahlen zeitlich definiert hineinbewegt wird.

5. Verfahren nach einem der vorhergehenden Ansprüche, **dadurch gekennzeichnet, dass** die Warenbahn von einem in der gleichen Bewegungsgeschwindigkeit vorlaufenden Endlossieb oder der Wandung einer Trommel abgedeckt wird und durch vorlaufende flüssigkeitsundurchlässige Teile, Linien, Flächen des Endlossiebes oder der Wandung der Trommel die Warenbahn im Bereich des auf dem unteren Vlies liegenden Gutes oder der zwischen den beiden Außenvliesen eingelegten endlichen Teile von den Wasserstrahlen unbeaufschlagt bleibt.

6. Verfahren nach Anspruch 5, **dadurch gekennzeichnet, dass** sich mit dem Endlossieb oder der Umfangsfläche der Trommel Flächenbereiche der Umfangsfläche vorwärts bewegen, die den Durchtritt des Wassers gegen die Warenbahn zeitlich und rhythmisch getaktet behindern und/oder vermindern.

7. Verfahren nach einem der vorhergehenden Ansprüche, **dadurch gekennzeichnet, dass** die abgelenkte, nicht die Warenbahn treffende Flüssigkeit sofort oberhalb der Warenbahn abgesaugt wird.

8. Verfahren nach einem der vorhergehenden Ansprüche, **dadurch gekennzeichnet, dass** die Wasserstrahlen aus nur teilweise über die Arbeitsbreite sich erstreckenden Düsenstreifen eines Düsenbalkens oder einer oder mehreren Düsen gegen die gegenüber dem Düsenbalken oder Düse vorlaufende Warenbahn gespritzt wird und der Düsenbalken oder die Düsen in einer Bewegung relativ zur Warenbahn oder umgekehrt gesteuert wird oder werden, um Teile, Linien, Flächen der Warenbahn im Bereich der mittleren, dreidimensionalen Schicht von den Wasserstrahlen unbeaufschlagt zu belassen, dagegen die Randbereiche dieser Flächen zu vernadeln zum Verbinden der dort nur zwei oder mehr aufeinanderliegenden Vliese.

9. Verfahren nach Anspruch 8, **dadurch gekennzeichnet, dass** der Austritt des Wassers aus der Düse oder dem Düsenbalken in Druck und/oder Menge in kurzen Abständen gesteuert wird.

10. Verfahren nach Anspruch 8, **dadurch gekennzeichnet, dass** mit einer punktuellen Fixierung auf diese Weise ein Vliesprodukt hydrodynamisch geheftet oder "genäht" wird.

11. Verfahren nach Anspruch 8 bis 10, **dadurch gekennzeichnet, dass** einzelne Düsen, Gruppen von Düsen oder Segmente eines breiteren Düsenbalkens über einzelne oder mehrere elektromechanisch, elektromagnetisch oder piezoelektrisch angesteuerte Ventile oder Ventilgruppen mit Flüssigkeit aus einer Hochdruckleitung versorgt werden.

12. Vorrichtung mit einem Düsenbalken an einer Vorrichtung zur Erzeugung von Flüssigkeitsstrahlen zur Strahlbeaufschlagung der Fasern von zumindest zwei entlang des Balkens mittels einer Trommel oder eines Endlosbandes geführten Bahnen, wie Faserbahn, Tissue, Gewebe oder Gewirke, der aus einem sich zumindest teilweise über die Arbeitsbreite der Bahn erstreckenden oberen Teil und einem unteren Teil besteht, wobei in dem oberen Teil über seine Länge eine im Querschnitt runde Druckkammer angeordnet ist, der die unter Druck stehende Flüssigkeit z. B. stirnseitig zugeführt ist, und an dem Unterteil ein Düsenblech mit den Öffnungen für die Düsen flüssigkeitsdicht gelagert ist, **dadurch gekennzeichnet**, der Düsenstreifen (4) über seine Länge Bereiche aufweist, die ohne Düsenlöcher (5, 5') versehen sind also den Durchtritt des Wassers oder die Entstehung von Wasserstrahlen über diese Bereiche verhindern und damit die endliche, dreidimensionale oder mittlere Schicht nicht zu vernadeln zum Verbinden der aufeinanderliegenden Vliese nur im Randbereich dieser Flächen.

13. Vorrichtung nach Anspruch 12, **dadurch gekennzeichnet, dass** der Düsenstreifen über seine Länge Bereiche mit einer unterschiedlichen Anzahl von Düsenlöchern oder unterschiedlichen Lochdurchmessern aufweist.

14. Vorrichtung mit einem Düsenbalken an einer Vorrichtung zur Erzeugung von Flüssigkeitsstrahlen zur Strahlbeaufschlagung der Fasern von zumindest zwei entlang des Balkens mittels einer Trommel oder eines Endlosbandes geführten Bahnen, wie Faserbahn, Tissue, Gewebe oder Gewirke, wobei der Düsenbalken aus einem sich über die Arbeitsbreite der Bahn erstreckenden oberen Teil und einem unteren Teil besteht, wobei in dem oberen Teil über seine Länge eine im Querschnitt runde Druckkammer angeordnet ist, der die unter Druck stehende Flüssigkeit z. B. stirnseitig zugeführt ist, und an dem Unterteil ein Düsenblech mit den Öffnungen für die Düsen flüssigkeitsdicht gelagert ist, **dadurch gekennzeichnet, dass** die Warenbahn (6) durch ein mitlaufendes, von den Flüssigkeitsstrahlen (5) beaufschlagtes Endlossieb (15) oder durch die Wandung einer Trommel abgedeckt ist und das Endlossieb (15) oder die Umfangsfläche der Trommel über die Arbeitsbreite verteilt flüssigkeitsundurchlässige Bereiche (16) aufweist, die die in der abgedeckten oder mittleren Schicht angeordneten endlichen Teile gegen die Beaufschlagung mit Wasserstrahlen abdecken.

15. Vorrichtung nach Anspruch 14, **dadurch gekennzeichnet, dass** die flüssigkeitsundurchlässigen Bereiche (16) Öffnungen (17) zum Durchtritt von Flüssigkeitsstrahlen aufweisen.

16. Vorrichtung mit mehreren Düsenbalken oder Düsen im Gehäuse an einer Vorrichtung zur Erzeugung von Flüssigkeitsstrahlen zur Strahlbeaufschlagung der Fasern von zumindest zwei entlang des Balkens mittels einer Trommel oder eines Endlosbandes geführten Bahnen, wie Faserbahn, Tissue, Gewebe oder Gewirke, der oder die aus einem sich über einen Teil der Arbeitsbreite der Bahn erstreckenden oberen Teil und einem unteren Teil besteht, wobei in dem oberen Teil eine Druckkammer angeordnet ist, der die unter Druck stehende Flüssigkeit zugeführt ist, und an dem Unterteil ein Düsenblech mit den Öffnungen für die Düsen flüssigkeitsdicht gelagert ist, **dadurch gekennzeichnet, dass** die Düsenbalken (1) oder die Düsen im Gehäuse nicht fixiert, sondern elektronisch mittels eines Computers in der Bewegungsbahn steuerbar gelagert sind, um Teile, Linien, Flächen der Warenbahn von den Wasserstrahlen unbeaufschlagt zu belassen, die Randbereiche dieser Flächen jedoch zu vernadeln zum Verbinden der dort nur zwei oder mehr aufeinanderliegenden Vliese.

17. Vorrichtung nach Anspruch 16, **dadurch gekennzeichnet, dass** die Düsenbalken oder Düsen in unterschiedlicher Richtung wie lotrecht oder schräg gegen die vorlaufende Warenbahn gerichtet sind.

18. Vorrichtung nach Anspruch 16, **dadurch gekennzeichnet, dass** die Düsenbalken oder Düsen in unterschiedlicher Höhe zur Warenbahn (6) oder zu den dreidimensionalen Produkten (7) angeordnet und/oder bewegbar sind.

19. Vorrichtung insbesondere nach Anspruch 16 bis 18 **dadurch gekennzeichnet, dass** der Austritt der Flüssigkeit an den Düsen mittels eines oder mehrerer Ventile gesteuert in Funktion setzbar ist.

20. Vorrichtung nach Anspruch 19 **dadurch gekennzeichnet, dass** die Ventile oder Ventilgruppen elektromechanisch, elektromagnetisch oder piezoelektrisch angesteuert sind.

21. Vorrichtung nach Anspruch 16 bis 20, **dadurch gekennzeichnet, dass** die Düsenbalken oder Düsen über Druckschläuche (18) an eine Druckpumpe oder einen Wasserbalken angeschlossen sind.

22. Vorrichtung nach einem der Ansprüche 16 bis 21 mit einer Absaugeinrichtung unterhalb der Warenbahn, **dadurch gekennzeichnet, dass** die Absaugeinrichtung mit der Düse bewegbar ist oder sich entlang der Bewegungsbahn der Düse erstreckt.

## Claims

1. Method for hydrodynamic compaction and/or consolidation and/or bonding in the case of at least two superposed nonwovens, tissue, woven fabrics or knitted fabrics using liquid jets emerging uniformly from a nozzle beam over a working width, in particular at a pressure of up to 1000 bar a liquid is sprayed, for example, from fine nozzle orifices arranged closely adjacent to one another in a row, e.g. from a nozzle strip (4) of a nozzle beam extending over a working width, towards the material web advancing towards the nozzle beam, **characterised in that** for the selective consolidation and/or binding, for inclusion of a finite, three-dimensional covered or central layer, in the case of liquid jets emerging unchanged from the elements producing the water jets, these are briefly prevented in parts from hitting the advancing material web and specifically in the area of the finite central layer in order to leave parts, lines, areas of the material web unexposed to the water jets but to needle the edge regions of these areas to bond the only two or more superposed nonwovens there.

2. The method according to claim 1, **characterised in that** when the liquid pressure acting against the nozzle strip remains continuously unchanged in the water beam, this is partly prevented from producing water jets over the length of the nozzle strip in order not to hit the advancing material web in this region with the finite, three-dimensional covered or central layer in order to leave parts, lines, areas of the material web unexposed to the water jets but to needle the edge regions of these areas to bond the only two or more superposed nonwovens there.

3. The method according to claim 1 or 2, **characterised in that** an object which changes the direction of motion of the water jets is moved in the jet direction of the water jets.

4. The method according to claim 3, **characterised in that** the object is moved into the direction of motion of the water jets in a time-defined manner.

5. The method according to any one of the preceding claims, **characterised in that** the material web is covered by an endless sieve which advances at the same speed or the wall of a drum and as a result of advancing liquid-impermeable parts, lines, areas of the endless sieve or the wall of the drum, the material web remains unexposed to the water jets in the area of the material lying on the lower nonwoven or the finite parts inserted between the two outer nonwovens.

6. The method according to claim 5, **characterised in that** surface regions of the circumferential surface move forwards with the endless sieve or the circumferential surface of the drum, which regions prevent and/or reduce the penetration of water to the material web in a temporally and rhythmically clocked fashion.

7. The method according to any one of the preceding claims, **characterised in that** the deflected liquid which does not hit the material web is immediately extracted above the material web.

8. The method according to any one of the preceding claims, **characterised in that** the water jets are sprayed towards the material web advancing towards the nozzle beam or nozzle from nozzle strips or a nozzle beam extending only partly over the working width of one or more nozzles, and the nozzle beam or the nozzles is or are controlled in a movement relative to the material web or conversely in order to leave parts, lines, areas of the material web in the area of the central three-dimensional layer unexposed to the water jets but to needle the edge regions of these areas to bond the only two or more superposed nonwovens there.

9. The method according to claim 8, **characterised in that** the emergence of the water from the nozzle or the nozzle beam is controlled in pressure and/or quantity at short distances.

10. The method according to claim 8, **characterised in that** a nonwoven product is hydrodynamically tacked or "sewn" by means of a localised fixing in this manner.

11. The method according to claim 8 to 10, **characterised in that** individual nozzles, groups of nozzles or segments of a broader nozzle beam are supplied with liquid from a high-pressure line by means of single or a plurality of electromechanically, electromagnetically or piezoelectrically controlled valves or valve groups.

12. A device comprising a nozzle beam on a device for producing liquid jets for acting upon fibres of at least two webs guided along the beam by means of a drum or an endless web, such as fibre web, tissue, woven fabric or knitted fabric, which consists of at least one upper part extending at least in part over the working width of the web and a lower part, wherein a circular-cross-sectional pressure chamber is disposed in the upper part over its length to which the pressurised liquid is supplied, e.g. at the front side and a nozzle sheet provided with the orifices for the nozzles is mounted in a liquid-tight fashion on the lower part, **characterised in that** the nozzle strip (4) has regions over its length which are provided without nozzle holes (5, 5') and thus prevent the passage of water or the formation of water jets over these regions and thereby do not needle the finite three-dimensional or central layer to bond the superposed nonwovens only in the edge regions of these surfaces.

13. The device according to claim 12, **characterised in that** the nozzle strip has regions with a different number of nozzle holes or different hole diameters over its length.

14. A device comprising a nozzle beam on a device for producing liquid jets for acting upon fibres of at least two webs guided along the beam by means of a drum or an endless web, such as fibre web, tissue, woven fabric or knitted fabric, wherein the nozzle beam consists of an upper part extending over the working width of the web and a lower part, wherein a circular-cross-sectional pressure chamber is disposed in the upper part over its length to which the pressurised liquid is supplied, e.g. at the front side and a nozzle sheet provided with the orifices for the nozzles is mounted in a liquid-tight fashion on the lower part, **characterised in that** the material web (6) is covered by a co-running endless sieve (15) which is acted upon by the liquid jets (5) or by the wall of a drum and the endless sieve (15) or the circumferential surface of the drum has liquid-impermeable regions (16) distributed over the working width which cover the finite parts disposed in the covered or central layer against the action by water jets.

15. The device according to claim 14, **characterised in that** the liquid-impermeable regions (16) have openings (17) for passage of liquid jets.

16. A device comprising a plurality of nozzle beams or nozzles in the housing on a device for producing liquid jets for acting upon fibres of at least two webs guided along the beam by means of a drum or an endless web, such as fibre web, tissue, woven fabric or knitted fabric, which consists of at least one upper part extending over a part of the working width of the web and a lower part, wherein a pressure chamber is disposed in the upper part over its length to which the pressurised liquid is supplied, e.g. at the front side and a nozzle sheet provided with the orifices for the nozzles is mounted in a liquid-tight fashion on the lower part, **characterised in that** the nozzle beams (1) or the nozzles are not fixed in the housing but are mounted such that they are controlled electronically by means of a computer in the movement path in order to leave parts, lines, areas of the material web in the area of the central three-dimensional layer unexposed to the water jets but to needle the edge regions of these areas to bond the only two or more superposed nonwovens there.

17. The device according to claim 16, **characterised in that** the nozzle beams or nozzles are directed in different directions such as perpendicular or at an angle to the advancing material web.

18. The device according to claim 16, **characterised in that** the nozzle beams or nozzles are arranged at different heights with respect to the material web (6) or to the three-dimensional products (7) and/or are movable.

19. The device, in particular according to claim 16 to 18, **characterised in** the exit of liquid to the nozzles can be set in operation controlled by one or more valves.

20. The device according to claim 19, **characterised in that** the valves or valve groups are driven electromechanically, electromagnetically or piezoelectrically.

21. The device according to claim 16 to 20, **characterised in that** the nozzle beam or nozzles are connected to a pressure pump or a water beam by means of pressure hoses (18).

22. The device according to any one of claims 16 to 21 comprising a suction device underneath the material web, **characterised in that** the suction device can be moved with the nozzle or extends along the movement path of the nozzle.

## Revendications

1. Procédé de densification et/ou consolidation et/ou liaison hydrodynamique dans le cas d'au moins deux non-tissés, tissus, textiles ou tricots superposés, au moyen de jets de liquide sortant régulièrement sur une largeur opérationnelle d'une poutre à buses, dans lequel, à une pression allant jusqu'à 1000 bar, un liquide est projeté depuis de fins orifices de buses disposés serrés les uns contre les autres en série, par exemple depuis une bande de buse (4) s'étendant sur une largeur opérationnelle d'une poutre à buse contre la bande de matière passant en face de la poutre à buses, **caractérisé en ce que**, pour consolider et/ou lier de manière sélective, pour envelopper une couche définitive, tridimensionnelle, couverte ou médiane tandis que des jets de liquide sortent sans changement des éléments générant les jets d'eau, on empêche partiellement pendant une brève durée ceux-ci de rencontrer la bande de matière passant devant, à savoir au niveau de la couche médiane définitive afin de laisser des parties, des lignes, des surfaces de la bande de matière exemptes d'une sollicitation par les jets d'eau mais d'aiguilleter les zones périphériques de ces surfaces afin de lier les seuls deux non-tissés ou davantage qui y sont superposés.

2. Procédé selon la revendication 1, **caractérisé en ce que**, tandis que la pression du liquide agit en continu sans changement dans la poutre à buses contre la bande de buses, on empêche partiellement cette poutre à buses de générer des jets d'eau afin de ne pas atteindre la bande de matière passant devant dans cette zone comprenant la couche définitive, tridimensionnelle, recouverte ou médiane afin d'exempter de toute sollicitation des parties, des lignes, des surfaces de la bande de matière par les jets d'eau mais d'aiguilleter les zones périphériques de ces surfaces afin de lier les seuls deux non-tissés ou davantage qui y sont superposés.

3. Procédé selon la revendication 1 ou 2, **caractérisé en ce que**, dans le sens de jet des jets d'eau, on déplace un objet qui modifie le sens de mouvement des jets d'eau.

4. Procédé selon la revendication 3, **caractérisé en ce que** l'objet est déplacé d'une manière définie dans le temps dans le sens de mouvement des jets d'eau.

5. Procédé selon une des revendications précédentes, **caractérisé en ce que** la bande de matière est recouverte par un crible sans fin avançant à la même vitesse de mouvement ou la paroi d'un tambour et que, grâce à des parties, des lignes, des surfaces imperméables au liquide avançant du crible sans fin ou de la paroi du tambour, la bande de matière reste exempte d'une sollicitation par les jets d'eau au niveau de la matière placée sur le non-tissé inférieur ou des parties définitives insérées entre les deux non-tissés extérieurs.

6. Procédé selon la revendication 5, **caractérisé en ce qu'**en même temps que le crible sans fin ou la surface circonférentielle du tambour se déplacent vers l'avant des zones surfaciques de la surface circonférentielle qui empêchent et/ou réduisent avec une synchronisation de temps et de rythme le passage de l'eau contre la bande de matière.

7. Procédé selon une des revendications précédentes, **caractérisé en ce que** le liquide dévié ne rencontrant pas la bande de matière est immédiatement aspiré au dessus de la bande de matière.

8. Procédé selon une des revendications précédentes, **caractérisé en ce que** les jets d'eau sont projetés depuis seulement des bandes de buses s'étendant partiellement sur la largeur opérationnelle d'une poutre à buses ou d'une ou plusieurs buses contre la bande de matière passant en face de la poutre à buses ou de la buse et que la poutre à buses ou les buses est ou sont contrôlées dans un mouvement par rapport à la bande de matière ou inversement afin de laisser des parties, des lignes, des surfaces de la bande de matière exemptes d'une sollicitation par les jets d'eau au niveau de la couche médiane tridimensionnelle mais par contre d'aiguilleter les zones périphériques de ces surfaces pour lier les seuls deux non-tissés ou davantage qui y sont superposés.

9. Procédé selon la revendication 8, **caractérisé en ce que** la sortie de l'eau hors de la buse ou de la poutre à buses est contrôlée à de brefs intervalles au niveau de la pression et/ou de la quantité.

10. Procédé selon la revendication 8, **caractérisé en ce qu'**un produit non-tissé est ainsi agrafé ou « cousu » par système hydrodynamique par fixation ponctuelle.

11. Procédé selon les revendications 8 à 10, **caractérisé en ce que** les buses individuelles, les groupes de buses ou les segments d'une poutre à buses plus large sont alimentés en liquide provenant d'une canalisation à haute pression par des soupapes ou groupes de soupapes à commande électromécanique, électromagnétique ou piézoélectrique isolées ou en plusieurs exemplaires.

12. Dispositif comportant une poutre à buses dans un dispositif de génération de jets de liquide pour projeter des jets sur les fibres d'au moins deux bandes guidées le long de la poutre au moyen d'un tambour ou d'une bande sans fin, comme une bande fibreuse, du tissu, du textile ou du tricot, qui est composé d'une partie supérieure s'étendant du moins partiellement sur la largeur opérationnelle de la bande et d'une partie inférieure, étant disposée dans la partie supérieure, sur sa longueur, une chambre de compression de section transversale ronde vers laquelle est acheminé le liquide sous pression, par exemple du côté frontal, et une tôle à buses comportant les orifices pour les buses s'appuyant de manière étanche aux liquides sur la partie inférieure, **caractérisé en ce que** la bande de buses (4) présente sur sa longueur des zones non pourvues de trous de buses (5, 5') et empêchant donc le passage de l'eau ou l'apparition de jets d'eau sur ces zones pour ne pas aiguilleter la couche définitive, tridimensionnelle ou médiane afin de lier les non-tissés superposés seulement dans la zone périphérique de ces surfaces.

13. Procédé selon la revendication 12, **caractérisé en ce que** la bande de buses présente sur sa longueur des zones comportant un nombre différent de trous de buses ou des diamètres différents de trous de buses.

14. Dispositif comportant une poutre à buses dans un dispositif de génération de jets de liquide pour projeter des jets sur les fibres d'au moins deux bandes guidées le long de la poutre au moyen d'un tambour ou d'une bande sans fin, comme une bande fibreuse, du tissu, du textile ou du tricot, dans lequel la poutre à buses est composée d'une partie supérieure s'étendant du moins partiellement sur la largeur opérationnelle de la bande et d'une partie inférieure, étant disposée dans la partie supérieure, sur sa longueur, une chambre de compression de section transversale ronde vers laquelle est acheminé le liquide sous pression, par exemple du côté frontal, et une tôle à buses comportant les orifices pour les buses s'appuie de manière étanche aux liquides sur la partie inférieure, **caractérisé en ce que** la bande de matière (6) est couverte par un crible sans fin (15) entraîné simultanément et sollicité par les jets de liquide (5) ou par la paroi d'un tambour et que le crible sans fin (15) ou la surface circonférentielle du tambour présente des zones imperméables au liquide (16) réparties sur la largeur opérationnelle et qui protègent les parties définitives disposées dans la couche recouverte ou médiane contre la sollicitation par des jets d'eau.

15. Dispositif selon la revendication 14, **caractérisé en ce que** les zones imperméables au liquide (16) présentent des orifices (17) pour le passage de jets de liquide.

16. Dispositif comportant plusieurs poutres à buses ou buses dans l'enceinte d'un dispositif de génération de jets de liquide pour projeter des jets sur les fibres d'au moins deux bandes guidées le long de la poutre au moyen d'un tambour ou d'une bande sans fin, comme une bande fibreuse, du tissu, du textile ou du tricot, qui est composé d'une partie supérieure s'étendant du moins partiellement sur la largeur opérationnelle de la bande et d'une partie inférieure, étant disposée dans la partie supérieure, sur sa longueur, une chambre de compression vers laquelle est acheminé le liquide sous pression, par exemple du côté frontal et une tôle à buses comportant les orifices pour les buses s'appuyant de manière étanche aux liquides sur la partie inférieure, **caractérisé en ce que** les poutres à buses (1) ou les buses ne sont pas fixées dans l'enceinte mais s'appuient dans la voie de mouvement de manière contrôlable par système électronique à l'aide d'un ordinateur afin de laisser des parties, des lignes, des surfaces de la bande de matière exemptes de toute sollicitation par les jets d'eau mais d'aiguilleter les zones périphériques de ces surfaces pour relier les seuls deux non-tissés ou davantage qui y sont superposés.

17. Dispositif selon la revendication 16, **caractérisé en ce que** les poutres à buses ou les buses sont orientées dans un sens différent, comme perpendiculairement ou à l'oblique, vers la bande de matière passant devant.

18. Dispositif selon la revendication 16, **caractérisé en ce que** les poutres à buses ou les buses sont disposées et/ou mobiles à une hauteur différente par rapport à la bande de matière (6) ou aux produits tridimensionnels (7).

19. Dispositif selon les revendications 16 à 18, **caractérisé en ce que** la sortie du liquide au niveau des buses peut être mise en marche de manière contrôlée au moyen d'une ou plusieurs soupapes.

20. Dispositif selon la revendication 19, **caractérisé en ce que** les soupapes ou groupes de soupapes sont contrôlés par système électromécanique, électromagnétique ou piézoélectrique.

21. Dispositif selon les revendications 16 à 20, **caractérisé en ce que** les poutres à buses ou les buses sont raccordées par des tuyaux à pression (18) à une pompe à pression ou à une poutre à eau.

22. Dispositif selon une des revendications 16 à 21, comprenant un dispositif d'aspiration en dessous de la bande de matière, **caractérisé en ce que** le dispositif d'aspiration est mobile avec la buse ou s'étend le long de la voie de mouvement de la buse.
